Europäisches Patentamt

European Patent Office

Office européen des brevets

(11) Publication number: **0 287 501 B1**

## EUROPEAN PATENT SPECIFICATION

(45) Date of publication of patent specification: **26.08.92**   (51) Int. Cl.⁵: **G01N 23/20**, G01N 23/00

(21) Application number: **88730047.3**

(22) Date of filing: **02.03.88**

(54) **Method for predicting life expectancy of materials.**

(30) Priority: **06.03.87 JP 50269/87**

(43) Date of publication of application:
**19.10.88 Bulletin  88/42**

(45) Publication of the grant of the patent:
**26.08.92 Bulletin  92/35**

(84) Designated Contracting States:
**DE FR GB**

(56) References cited:
**US-A- 4 404 682**

(73) Proprietor: **MITSUBISHI JUKOGYO KABUSHIKI KAISHA**
**5-1, Marunouchi 2-chome Chiyoda-ku**
**Tokyo 100(JP)**

Proprietor: **NIPPON STEEL CORPORATION**
**6-3 Otemachi 2-chome Chiyoda-ku**
**Tokyo 100(JP)**

(72) Inventor: **Nishida, Takashi Takasago Techni-cal Institute**
**Mitsubishi Jukogyo K.K. 1-1, Shinkawa**
**2-chome Arai**
**Takasago Hyogo Pref.(JP)**
Inventor: **Tashimo, Masanori Kobe Shipyard &**
Engine
**Works, Mitsubishi Jukogyo K.K. 1-1,**
**Wadasaki-cho**
**1-chome Hyogo-ku Kobe Hyogo Pref.(JP)**
Inventor: **Ogino, Masaaki Kobe Shipyard &**
**Engine**
**Works, Mitsubishi Jukogyo K.K. 1-1,**
**Wadasaki-cho**
**1-chome Hyogo-ku Kobe Hyogo Pref.(JP)**
Inventor: **Nakazawa, Takanori-R&D**
**Laboratories-II**
**Nippon Steel Corporation 10-1, Fuchinobe**
**5-chome**
**Sagamihara Kanagawa Pref.(JP)**
Inventor: **Funaki, Syuichi R&D Laboratories II**
**Nippon Steel Corporation 10-1, Fuchinobe**
**5-chome**
**Sagamihara Kanagawa Pref.(JP)**
Inventor: **Kuroki, Tuneo Osaka Sales Office**
**Nippon Steel Corporation 2-4, Nakanoshima**
**3-chome**
**Osaka Osaka(JP)**

(74) Representative: **Meissner, Peter E., Dipl.-Ing.**
**et al**
**Patentanwaltsbüro Meissner & Meissner,**
**Herbertstrasse 22**
**W-1000 Berlin 33(DE)**

# Description

## FIELD OF THE INVENTION AND RELATED ART STATEMENT:

The present invention relates to a method for predicting life expectancy of parts made of crystalline materials.

There exists a conventional method using a strain gauge to predict how much of lifetime has elapsed for all types of parts made of crystalline materials. This method using a strain gauge measures stress acting on a part through changes in resistance of a resistor attached to the surface thereof.

However, this method using a strain gauge, after all, measures stress in an indirect manner and the results cannot be related directly to lifetime already spent.

Parts under stress develope irreversible strain according to forces acting on them and eventually breaks down. There is a certain relationship between accumulated strain and lifetime already spent. Therefore, if the amount of irreversible strain in a part itself can be measured, the life expectancy of the part may be predicted from a predetermined correspondence between the amount of strain and the spent lifetime.

However, this amount of strain is extremely small and it has not been possible to measure it.

## OBJECT AND SUMMARY OF THE INVENTION:

In the present invention a crystal with relatively more information is selected for the measurement of small strain on a crystalline scale by polishing a sample surface and analysing electron channeling contrast (abbreviated as ECC hereafter) images and the like of surface crystals obtained by using a scanning electron microscope (abbreviated as SEM hereafter) and the like. Next, an electron channeling pattern (abbreviated as ECP hereafter) image of the selected crystal is obtained, and from the quality of this image the amount of strain accumulated in the crystal is measured. From a preestablished relationship between strain and quality of ECP images for the similar materials the life expectancy of the part can be estimated together with some consideration on conditions under which the part has been used. It is an object of the present invention to provide an entirely new method, as described above, for predicting life expectancy of parts.

## BRIEF DESCRIPTION OF DRAWINGS:

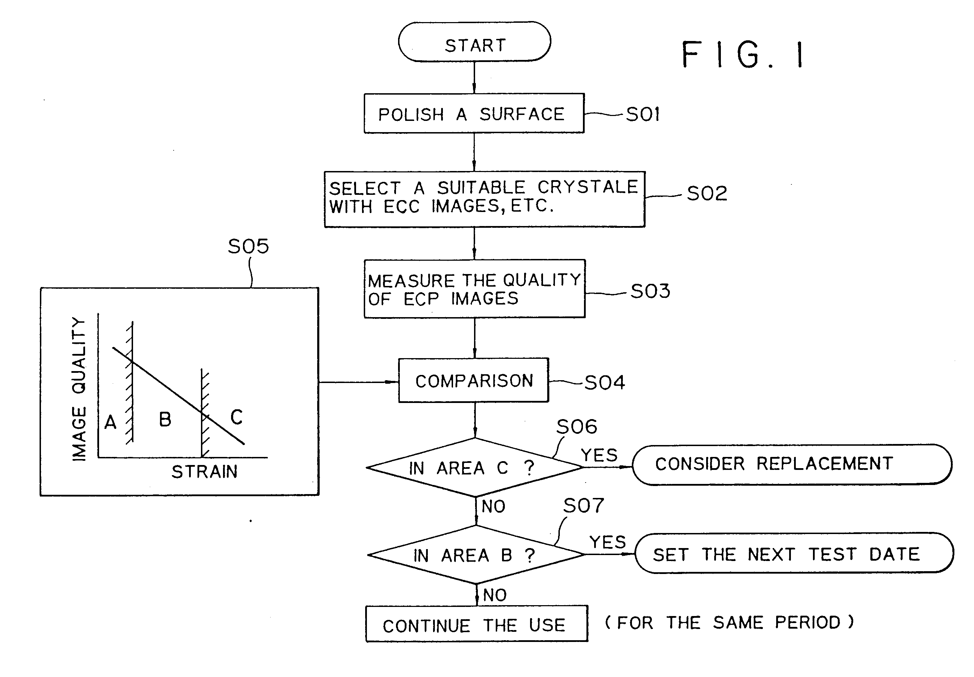

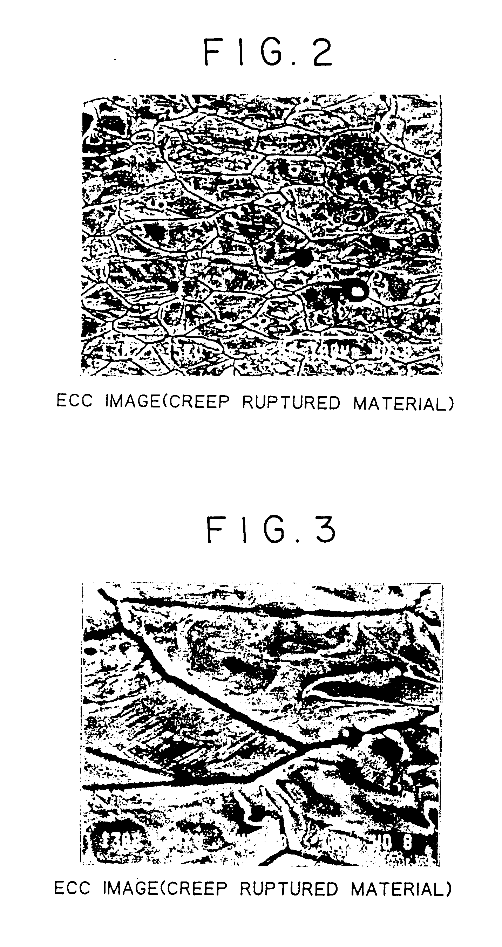

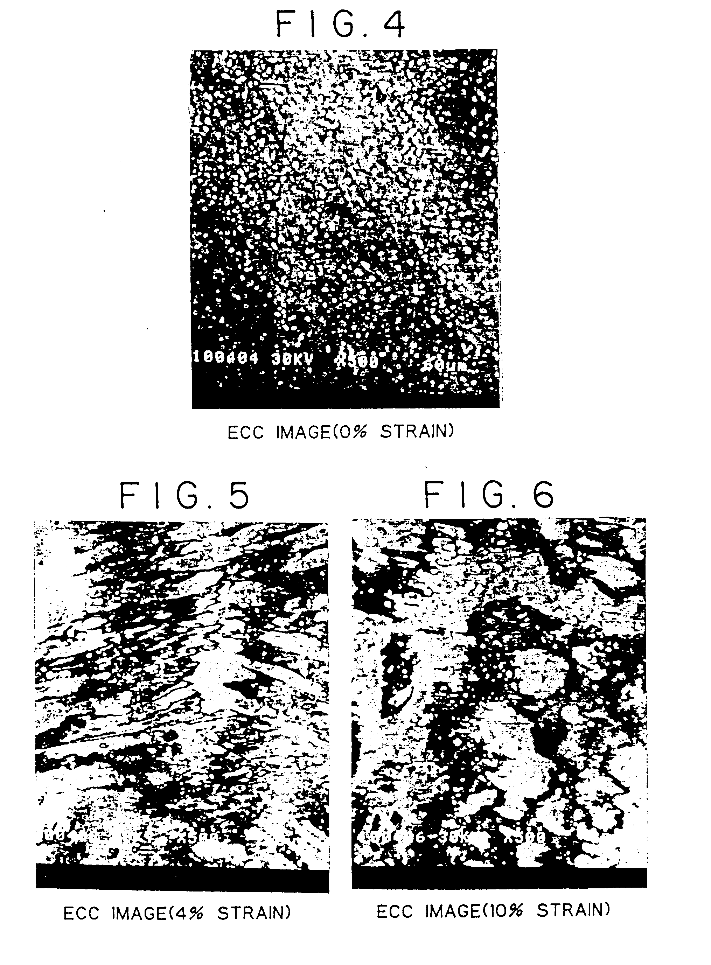

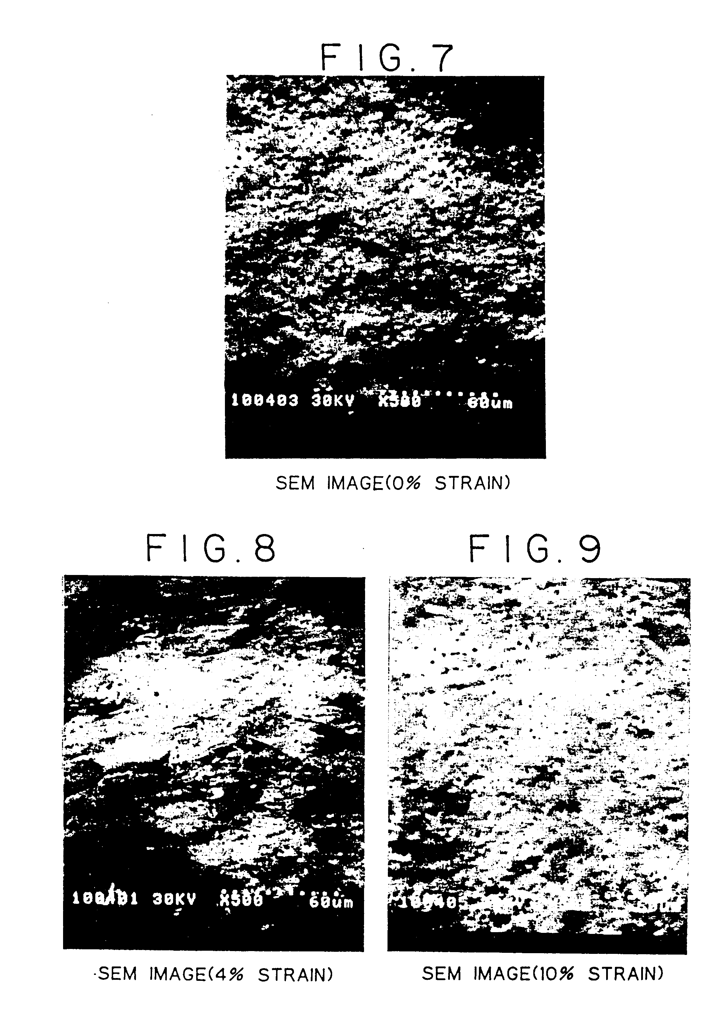

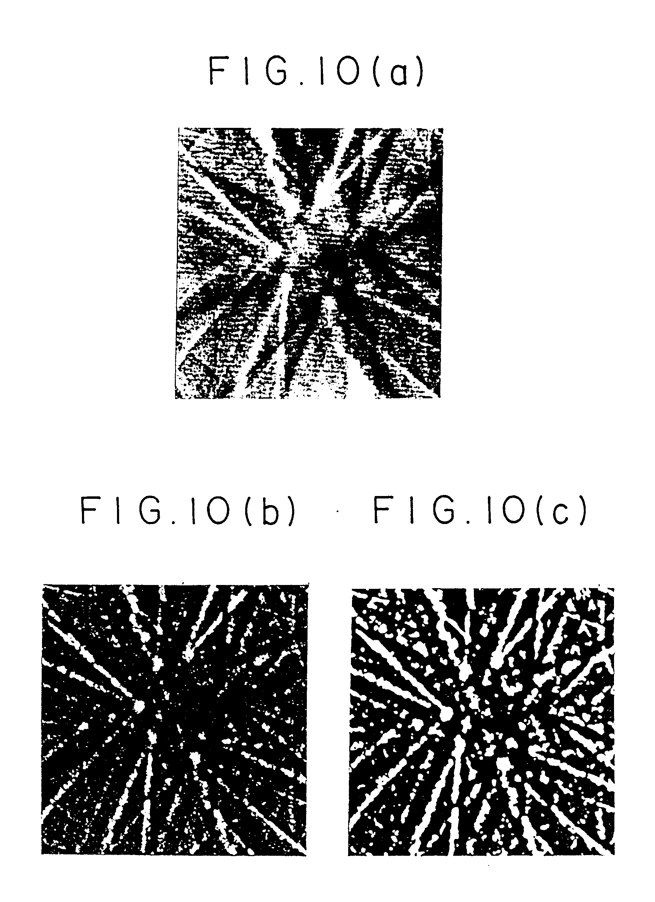

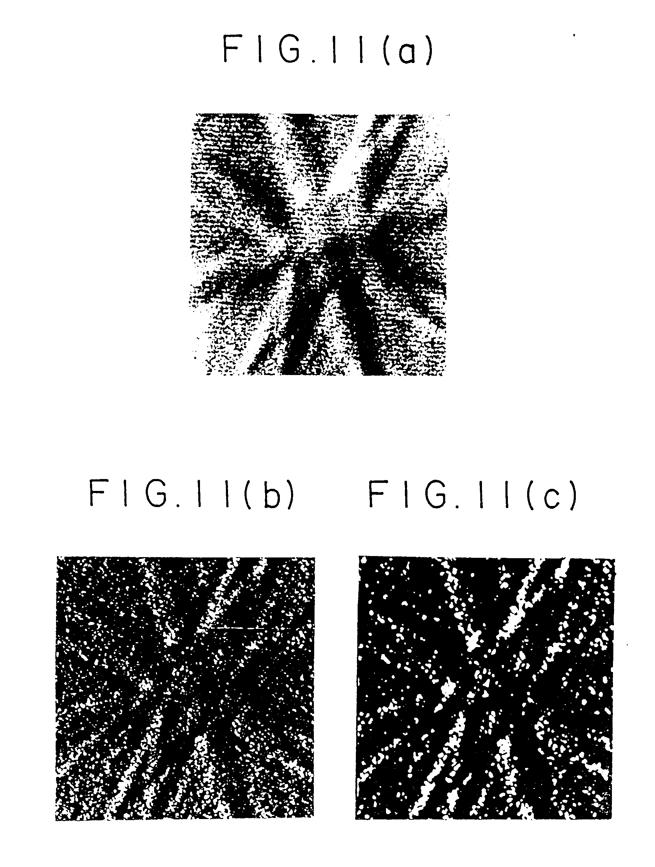

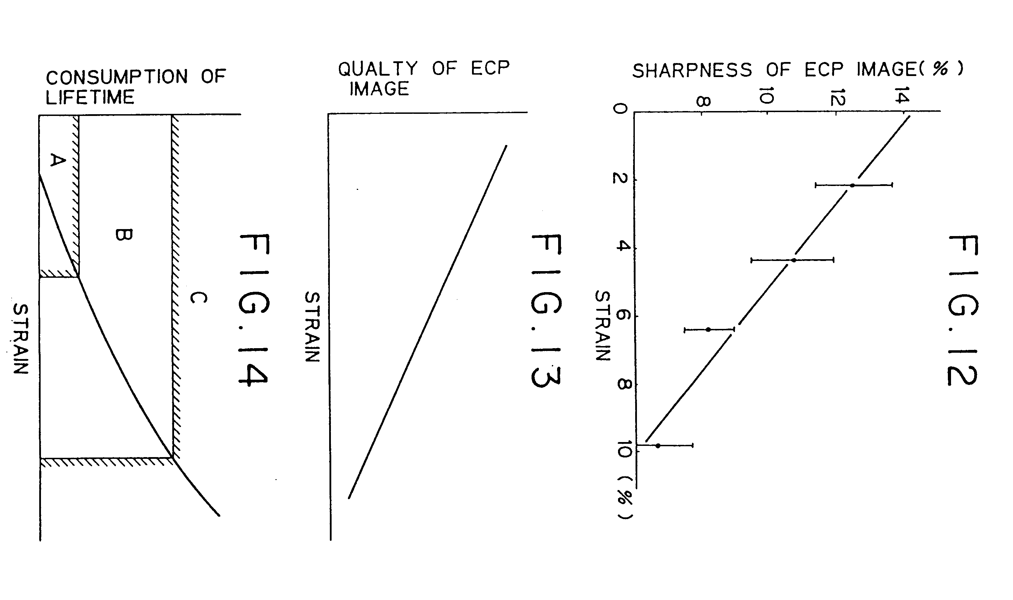

The following drawings show an embodiment of the present invention: FIG. 1 shows a flow chart of treatment procedures of the present invention; FIGS. 2 to 6 show ECC images in an embodiment of the present invention; FIGS. 7 to 9 show SEM images in an embodiment of the present invention; FIGS. 10 and 11 show EPC images in an embodiment of the present invention; FIG. 12 shows relationship between strain and sharpeness of ECP images of a part obtained from FIGS. 10 and 11; and FIG. 14 shows relationship between a lifetime ratio and strain of a crystal.

## DETAILED DESCRIPTION OF THE PREFERRED EMBODIMENT:

In the following, with reference to the drawings, an embodiment of the present invention will be explained.

FIG. 1 shows the flow of the measurement process in an embodiment of the present invention. As shown in Step SO1 a measuring point on a part is finely polished by a method of chemical or electrolytic polishing. Or a part can be polished and coated with a protective layer prior to use. During the course of its use, the part is removed from the system, and its surface is polished as above, or the protective layer is removed fromit. ECC images of the polished surface are obtained using an SEM (scanning electron microscope). Then, as shown in Step SO2 a suitable crystal for the following analysis is selected using ECC images and the like.

FIGS. 2 and 3 show examples of ECC images. In the case of a ruptured material shown in FIG. 3, for example, a crystal with more information such as the one with glide lines is selected. This can also be done with SEM images.

FIGS. 4 to 6 show ECC images of single-crystal rolled silicon steel, and

FIGS. 7 to 9 show SEM images of the same material, each cold rolled at a different rolling rate. The selection of a crystal is particularly easy with ECC images because magnification can be continuously varied from the order of 10 times to that of 10,000 times with ECC.

By selecting a crystal with more information it becomes possible to measure a maximum strain locally present in a part and therefore predict its life expectancy with higher accuracy incorporating mechanisms of rupture. Namely, because of different anisotropy of each crystal, crystals with differing amounts of deformation coexist in multicrystalline materials. Rupture of a material is often triggered by cracking of a particular crystal or crystal granule. Therefore, the strain of a crystal with particularly large amounts of fatigue and creep, a crystal that probably will first crack, is the key factor in predicting life expectancy of the whole material.

Previously some attempts were made to find life expectancy of materials by measuring strain using X-ray diffraction and the like. A method for foreseeing the residual life of a structural member by using X-ray diffraction is disclosed in US-A-4404682. But with this type of methods it has not been possible to single out a particular crystal with more fatigue and measure its strain. Instead, only an average value of strain can be obtained for a rather large area of a surface. That is to say, with an X-ray method although a diffraction plane corresponding to averaged strain is emphasized, a lattice plane of a particularly fatigued crystal cannot be observed very well with small signal strength. On the contrary, in the present invention, selecting a particular crystal enables one to predict life expectancy with the highest accuracy.

After selecting a suitable crystal in Step SO2, an ECP image of the selected crystal is made and the quality of the image is measured in a quantitative fashion in Step SO3. FIGS. 10 and 11 are to show the manner in which the quality of an image is measured. FIG. 10 shows an ECP image of a silicon steel plate with no strain, and FIG. 11 that of a silicon steel plate with 10% strain. In the present embodiment, an ECP image (FIGS. 10(a) and 11-(a)) is first obtained for a selected crystal as follows: A rocking coil for generating ECP's is attached to a SEM, and a parallel incident electron beam is fixed at a point by scanning the beam source and varying the angle of incidence at the same time. By this beam rocking the varying amount of diffracted electrons according to the Bragg condition of a surface crystal is recorded and displayed on a CRT as ECP images shown in FIGS. 10(a) and 11(a).

Next, the ECP images thus obtained are fed into an image processor, and the original images are enhanced by a filter. Then, sharp edgy parts of the enhanced images are further emphasized and extracted with a low-pass filter, and FIGS. 10(b) and 11(b) are obtained. From these images noise is removed with a median filter and they are binary coded for sharp parts of the images to yield FIGS. 10(c) and 11(c). These images are analyzed to determine their sharpness as quantitative information of image quality by finding an occupying ratio of the binary coded images. Namely, the sharpness here is expressed as the ratio of sharp areas in the original image to quantify the loss of sharpness of the ECP image as strain increases. If the original ECP image is sharp, then the sharpness measured in the above fashion increases.

In the present embodiment this sharpness is used as an indicator. If a different image processing technique is used, however, such sharpness may be different for the exactly same material. So the correspondence between strain and this sharpness has to be always made under the same condition.

For the material with no strain the sharpness is found to be 14% from FIG. 10(c), for example. Also, the sharpness of the material with 10% strain, shown in FIG. 11(c), is 6%. The sharpness thus obtained is compared to a normalized curve of Step SO5 in Step SO4 of FIG. 1.

FIG. 12 shows the relationship between strain and the sharpness thus measured three time for each strength of strain.

FIG. 13 shows a predetermined normalized curve of sharpness as a function of strain obtained by repeating the procedure explained above for test materials under accelerating conditions. Also, a predetermined relationship between strain of a crystal and spent lifetime can be obtained by studying data for the above normalized curve and is shown in FIG. 14. This relationship is obtained, for example, from test pieces for fatigue, creep and creep fatigue before and after rupture.

In FIG. 14 Area C is where replacement is required, and Area B is where reexamination should be scheduled after a period of time that depends on the measured amount of strain. Also, if the strain is in Area A it is unnecessary to test the part again until the same period of time as between the present and previous tests elapse.

At Step SO6 it is determined whether the measured strain falls in Area C of FIG. 14. If the answer is yes the part is ready to be replaced. If no, it is judged whether the strain is in Area B at Step SO7. If the answer here is yes the next test is scheduled considering the conditions under which the part is used together with the position of the strain in FIG. 14. Further, if the strain is not in Area B either, it is in Area A and the part can be used for the same period of time as between the present and previous tests.

When a part to be tested is large and it is difficult to use a SEM, a thin plate or foil of a material whose properties are already known can be attached to the large part so that forces acting on the part is passed onto the thin plate or foil. This thin plate or foil is tested to find life expectancy of the part. Also, this can be conveniently done while the part itself is in use.

As described above in detail the present invention enables one to measure the amount of strain from quantified quality of ECP images of a selected crystal on a finely polished surface of a part in an equipment to be tested. Therefore, it becomes possible to predict life expectancy of an equipment with high accuracy at an early stage by finding how much the part is damaged and determine when to start considering replacement of parts and repair of the equipment.

**Claims**

1. A method for predicting life expectancy of parts made of crystalline materials comprising:
   a polishing means for finely polishing, as a pretreatment, a surface of a part that is put under stress;
   a selection means for selecting a crystal with a large amount of information by processing an electron beam diffracted from said polished surface of said part with the above polishing means to obtain an electron channeling contrast (ECC) image;
   an imaging means for obtaining an electron channeling pattern (ECP) image of the crystal with a large amount of information selected by the above selection means; and
   a prediction means for predicting the consumption of lifetime of said part up to the time of measurement from the quality of electron channeling pattern image obtained by the above imaging means and using predetermined relationships between the image quality and strain in crystals and between strain and spent lifetime.

2. The method for predicting life expectancy described in Claim 1 further comprising an image processing means for binary coding and quantifying, together with filters, the quality of said electron channeling pattern (ECP) image obtained by the above imaging means.

3. The method for predicting life expectancy described in any of the above claims further comprising a relating means for relating the measured strain to the timing of replacement and reexamination of said part.

**Patentansprüche**

1. Verfahren zur Vorhersage der Lebenserwartung von Teilen aus kristallinem Material mit:
   einem Poliermittel zum Feinpolieren als eine Vorbehandlung, einer Oberfläche des Teils, die unter eine Spannung gesetzt wird;
   einem Auswahlmittel zum Auswählen eines Kristalls mit einer großen Menge an Informationen durch Verarbeitung eines von der durch die obigen Poliermittel polierten Oberfläche des Teils gebeugten Elektronenstrahls, um ein Elektronen kanalisierendes Kontrastbild "ECC" (electron channeling contrast image) zu erzielen;
   einem Bildentwurfsmittel zur Erzielung eines Elektronen kanalisierenden Schemabildes "ECP" (electron channeling pattern image) des Kristalls mit einer großen Menge an Informationen, welches durch die oben genannten Auswahlmittel ausgewählt wurde; und
einem Vorhersagemittel zum Vorhersagen des Verbrauchs an Lebensdauer des Teils bis zum Zeitpunkt der Qualitätsmessung des durch das obengenannte Bildentwurfsmittel erzielten elektronenkanalisierenden Schemabildes und Verwendung bestimmter vorbestimmter Beziehungen zwischen der Bildqualität und der Spannung im Kristall und zwischen der Spannung und der verbrauchten Lebensdauer.

2. Verfahren zur Vorhersage der Lebenserwartung nach Anspruch 1, dadurch gekennzeichnet, daß es weiterhin ein Bildverarbeitungsmittel umfaßt, um zusammen mit Filtern die Qualität des Elektronen kanalisierenden Schemabildes "ECP", welches durch das oben genannte Bildentwurfsmittel erzielt wurde, binär zu verschlüsseln und zu quantifizieren.

3. Verfahren zur Vorhersage der Lebenserwartung nach einem der vorhergehenden Ansprüche, dadurch gekennzeichnet, daß es weiterhin ein Beziehungsmittel umfaßt, um die gemessene Spannung mit der Zeitpunkteinstellung zum Ersetzen und Wiederüberprüfen des Bauteils in Beziehung zu setzen.

**Revendications**

1. Procédé pour prévoir l'espérance de vie de pièces réalisées à partir de matériaux cristallins, comprenant :
   - des moyens de polissage pour finement polir, en tant que prétraitement, une surface d'une pièce sous contrainte ;
   - des moyens de sélection pour choisir un cristal ayant une grande quantité d'informations en traitent un faisceau d'électrons diffracté à partir de ladite surface polie de ladite pièce avec lesdits moyens de polissage pour obtenir une image de contraste à cheminement préférentiel des électrons (ECC) ;
   - des moyens de représentation pour obtenir une image de structure à cheminement préférentiel des électrons (ECP) du cristal ayant une grande quantité d'informations, choisi par lesdits moyens do sélection ; et
   - des moyens de prédiction pour prévoir la consommation de durée de vie de ladite pièce jusqu'au moment de la mesure à partir de la qualité de l'image de structure à cheminement préférentiel des électrons obtenue par lesdits moyens de représentation, et utilisant des relations prédéterminées entre la qualité de l'image et la déformation dans des cristaux et entre la déformation et la durée de vie écoulée.

2. Procédé pour prévoir l'espérance de vie selon la revendication 1, comprenant de plus des moyens de traitement d'images pour coder de façon binaire et quantifier, avec des filtres, la qualité de ladite image de structure à cheminement préférentiel des électrons (ECP) obtenue par lesdits moyens de représentation.

3. Procédé pour prévoir l'espérance de vie selon l'une quelconque des revendications ci-dessus, comprenant de plus des moyens pour faire correspondre la déformation mesurée au cycle de remplacement et de réexamen de ladite pièce.

FIG. 1

START

POLISH A SURFACE — SO1

SELECT A SUITABLE CRYSTALE WITH ECC IMAGES, ETC. — SO2

MEASURE THE QUALITY OF ECP IMAGES — SO3

SO5

COMPARISON — SO4

IMAGE QUALITY

A    B    C

STRAIN

SO6

IN AREA C ? — YES → CONSIDER REPLACEMENT

NO

SO7

IN AREA B ? — YES → SET THE NEXT TEST DATE

NO

CONTINUE THE USE    ( FOR THE SAME PERIOD )

EP 0 287 501 B1

# F I G. 2

ECC IMAGE(CREEP RUPTURED MATERIAL)

# F I G. 3

ECC IMAGE(CREEP RUPTURED MATERIAL)

# FIG. 4

ECC IMAGE(0% STRAIN)

# FIG. 5

ECC IMAGE(4% STRAIN)

# FIG. 6

ECC IMAGE(10% STRAIN)

# FIG.7

SEM IMAGE(0% STRAIN)

# FIG.8

·SEM IMAGE(4% STRAIN)

# FIG.9

SEM IMAGE(10% STRAIN)

FIG.10(a)

FIG.10(b) · FIG.10(c)

# FIG.11(a)

# FIG.11(b)

# FIG.11(c)

# F I G . 12

SHARPNESS OF ECP IMAGE(%)

STRAIN

# F I G . 13

QUALTY OF ECP IMAGE

STRAIN

# F I G . 14

CONSUMPTION OF LIFETIME

C

B

A

STRAIN